# EUROPEAN PATENT SPECIFICATION

(11) **EP 2 237 804 B1**
(45) Date of publication and mention of the grant of the patent: **10.04.2013**
(21) Application number: 08798732.7
(22) Date of filing: 27.08.2008
(51) Int. Cl.: B01D 15/20, B01J 20/34, B01J 20/32, A61L 2/04, A61L 2/07, B01J 20/00

(54) **METHOD FOR STERILIZATION OF CHEMICALLY ACTIVATED SOLID SUPPORT MATERIALS**
VERFAHREN ZUR STERILISATION VON CHEMISCH AKTIVIERTEN FESTEN STÜTZMATERIALIEN
PROCÉDÉ DE STÉRILISATION DE MATÉRIAUX DE SUPPORT SOLIDES ACTIVÉS CHIMIQUEMENT

(30) Priority: 21.12.2007 US 15686
(43) Date of publication of application: 13.10.2010
(73) Proprietor: GE Healthcare Bio-Sciences AB, 751 84 Uppsala (SE)
(72) Inventor: NEU, Henrik, S-751 84 Uppsala (SE); ADIELSSON, Patrik Emanuel, S-751 84 Uppsala (SE)
(74) Representative: Kilander, Ebba Annika
(86) International application number: PCT/US2008/074366
(87) International publication number: WO 2009/082515

(56) References cited:
- WO-A-95/31727
- WO-A-2006/096116

## Description

### Field of the Invention

The present invention relates to a method for sterilization of different materials, especially sensitive materials. In particular, the invention relates to a method for the sterilization of chemically activated solid support materials.

### Background of the Invention

The manufacture of biopharmaceuticals, particularly drugs based on bioactive molecules such as proteins, peptides and nucleic acids, requires the production and purification of these molecules on an industrial scale. In particular, the increasing demand for monoclonal antibodies (MAbs) as biopharmaceutical products has promoted the development of cell cultures with high expression levels and, as a consequence, the demand for more efficient purification processes has increased. For example, monoclonal antibodies are revolutionizing the treatment of many illnesses and they have become one of the main indicators of the direction drug treatments are moving. The latest estimates of the European monoclonal antibody therapeutics market arrive at a figure of $11.4 billion (€8.7 billion) by 2011. This increased demand has necessitated the use of large, advanced chromatography systems comprising columns packed with separations media such as SEPHAROSE^{™}, MABSELECT^{™}, SOURCE^{™} and CAPTO^{™} (GE Healthcare).

During chromatographic separation of biopharmaceuticals, it is of critical importance to ensure that the process is conducted under sterile conditions and potentially harmful contaminants are removed from the system before use. Contamination with bacteria and other microbes is an often encountered problem within many biotechnological and biomedical applications. Various agents are known for their ability to inactivate and/or destroy microbial populations, for example, sodium hydroxide, peracetic acid, phosphoric acid, ethylene oxide, chlorine dioxide and benzyl alcohol. However, disinfection of columns and chromatographic media is cumbersome, particularly disinfection of proteinaceous media, i.e. chromatographic media provided with proteinaceous ligands, such as various affinity chromatography media. The most effective disinfectants and sanitation reagents such as strong acids or alkalis, quaternary ammonium compounds, halogen-containing compounds, oxidizing agents, and phenols and related compounds are considered harmful to most chromatography media particularly to proteinaceous affinity media. In addition, sterilization methods, such as gamma irradiation and autoclaving, are also considered to have large deleterious effects on those media. Furthermore, many sanitation or sterilization methods involving acids or alkalis, quaternary ammonium compounds, halogen-containing compounds, oxidizing agents, and phenols and related compounds are harmful and/or toxic.

US 5817528 (Böhm, W. et al) describes a method for producing a sterile and pyrogen-free column containing a matrix material to which a protein is coupled. Suitably, the protein coupled to the column is *Staphylococcus aureus* Protein A, or Streptococcus Protein G, or the protein may be an antibody such as anti-human LDL immunoglobulin or anti-human Ig immunoglobulin. The method provides a column matrix material such as an agarose which is chemically activated, using CNBr/triethylamine or using 1,1'-carbonyldiimidazole. However, this method is quite cumbersome as the agarose is first sterilized and the subsequent activation and coupling steps has to be performed under sterile conditions.

WO 2006/096116 and WO 95/31727 also deal with sterilisation of chromatography column media using steam or super - heated aqueous liquid.

Methods are required for the efficient sterilization of sensitive material, such as pre-activated chromatographic media, such that only the ligand coupling step needs to be performed under aseptic or sterile conditions.

### Summary of the Invention

The present invention provides a new sterilization method for bacterial and/or viral contamination of activated solid support material, particularly in chromatographic separation media used in the purification of biopharmaceutical materials. The inventors have found that pre-activated solid support having particular chemical groups is surprisingly resistant to denaturation by sterilization by autoclaving.

Thus, in a first aspect, there is provided a method for sterilization of an aldehyde-activated solid support, the method comprising exposing the solid support to pressurized steam at a temperature of between 121°C and 135°C.

In one embodiment, the solid support is exposed to pressurized steam under a pressure in the range from 2 bars to 35 bars. Preferably, the solid support is exposed to pressurized steam under a pressure in the range from 30-35 bars, more preferably under a pressure in the range from 34-35 bars.

In one embodiment, the solid support is exposed to pressurized steam for a time period in the range from 10 to 60 minutes.

In a particular embodiment, the aldehyde-activated solid support is sterilized by exposure to pressurized steam at a temperature of 121°C and 34 bars for 15 minutes.

In one embodiment, the solid support to be sterilized is a chromatography matrix. Optionally, the chromatography matrix is contained in a chromatography column or supported on a filter.

In one embodiment, the solid support comprises an aldehyde-activated agarose matrix.

In another aspect, there is provided a sterile aldehyde-activated solid support, produced by a process comprising (a) providing an aldehyde-activated solid support; and (b) exposing the solid support to pressurized steam at a temperature of between 121°C and 135°C.

In one specific embodiment, the solid support comprises an aldehyde-activated agarose matrix.

In another embodiment, the solid support is a chromatography matrix.

There is provided a sterile chromatographic separation medium, comprising a ligand coupled to a sterile chromatography matrix produced by embodiments of the current invention.

In another aspect, there is provided a method for producing a sterile chromatographic separation medium, comprising coupling a ligand under aseptic conditions to the sterile chromatography matrix produced by embodiments of the current invention.

### Detailed Description of the Invention

The present invention provides a new and efficient method for sterilization of pre-activated solid support materials. These materials are particularly useful as chromatography media or components for medical devices. In particular, the invention relates to a method for bacterial and viral inactivation of solid support materials pre-activated by aldehyde groups, such as aldehyde activated agarose matrix. The method is based on an exposure of the solid support materials to pressurized steam at a temperature of between about 121°C and about 135°C, more particularly between about 121°C and about 123°C. In a particularly preferred embodiment, the solid support materials are exposed to pressurized steam at a temperature of 121°C.

Generally, moist heat sterilization by autoclaving refers to heating a material in an autoclave (e.g. gravity displacement apparatus) under a pressure of at least 2 bars to achieve a temperature of between about 121°C and about 135°C. In the sterilization process, microorganisms are killed by heating in the presence of moisture and elevated pressure. See for example, "Understanding the Operation & Validation of Autoclaves: A Practical Approach", Reeks, B., BDR Publishing (September 1999). The sterilization period required is dependent on both the temperature and the size of the sample to be sterilized and can be in the range from 10 to 60 minutes. As the temperature and pressure are increased, the time required to achieve complete sterilization can normally be reduced, as shown in Table 1.

**Table 1**

| Temperature (°C) | Time (minutes) | Pressure (bars, abs) |
|---|---|---|
| 121 - 124 | 15 | 2.01 |
| 126 - 129 | 10 | 2.4 |
| 134 - 137 | 3 | 3.05 |

Conventionally, pre-activated solid support materials including chromatography matrices are not sterilized by autoclaving because they are regarded as unstable under moist heat sterilization conditions. Thus, these solid support materials are usually sanitized (reduction of the numbers of microbial contaminants to an acceptable level), rather than by the complete sterilization. To test the method for sterilizing these solid support materials described herein, the inventors have utilized the elevated temperatures and pressures obtainable with a Getinge GE EC-1 autoclave with a PACS 2000 software. The instrumentation can be programmed for temperature, time and pressure. The sterilization was performed under standard conditions with a temperature between 121,1-123 degrees for 15 minutes. As used herein, the term "chromatography matrix (or matrices)" refers to a stationary or particulate phase which is effective to bind (i.e. adsorb) a target material under selected mobile phase conditions, and to release the target material under other selected mobile phase conditions. The term "pre-activated chromatography matrix (or matrices)" refers to chromatography matrix which contains certain chemical groups for the coupling of ligands. The process described herein is particularly suitable for sterilizing pre-activated chromatography matrix. A preferred chromatography matrix is aldehyde activated agarose matrix, such as SEPHAROSE^{™} or CAPTO^{™}.

The solid support material according to the invention can be of any suitable well-known kind. In the most preferred embodiment, the solid support is porous agarose beads. In one embodiment, the solid support material is useful as a component for medical devices. In another embodiment, the solid support is a chromatography matrix.

A conventional chromatography matrix is often of organic nature and based on polymers that expose a hydrophilic surface to the aqueous media used, i.e. expose hydroxy (-OH), carboxy (-COOH), carboxamido (-CONH₂, optionally in N-substituted forms), amino (-NH₂, optionally in substituted form), oligo- or polyethyleneoxy groups on their external and, if present, also on internal surfaces. In one embodiment, the polymers may, for instance, be based on polysaccharides, such as dextran, starch, cellulose, pullulan, agarose etc, which advantageously have been cross-linked, for instance with bis-epoxides, epihalohydrins, 1,2,3-trihalo-substituted lower hydrocarbons, to provide a suitable porosity and rigidity.

Alternatively, the solid support is based on synthetic polymers, such as polyvinyl alcohol, polyhydroxyalkyl acrylates, polyhydroxyalkyl methacrylates, polyacrylamides, polymethacrylamides etc. In case of hydrophobic polymers, such as materials based on divinyl and monovinyl-substituted benzenes, the surface of the matrix is often hydrophilized to expose hydrophilic groups as defined above to a surrounding aqueous liquid.

In yet another alternative, the solid support may be of inorganic nature, e.g. silica, zirconium oxide, etc.

In a further embodiment, the solid support is in another form such as a surface, a chip, capillaries, or a filter.

The solid support matrix generally requires an activation step, thereafter ligands are attached to the support via conventional coupling techniques utilizing. Coupling of ligands via epoxide, CNBr, NHS and aldehyde groups are all well known methods. Hermanson, G.T., A.K. Mallia, and P.K. Smith, Immobilization of ligands, in Immobilized Affinity Ligand Techniques. 1992, Academic Press. A spacer group can be introduced between the support and the ligand, thereby improving the availability of the ligand and facilitating the chemical coupling of the ligand to the support.

The inventors have found unexpectedly that while certain CNBr and NHS activated solid support materials could not withstand sterilization by autoclavation, aldehyde activated materials are stable and are not adversely affected by autoclavation.

The method is therefore particularly suitable for the sterilization of solid support material pre-activated with aldehyde groups, such as aldehyde activated agarose matrix. For a review of aldehyde activation and ligand coupling, see: Hermanson, G.T., A.K. Mallia, and P.K. Smith, Immobilization of Ligands, in Immobilized Affinity Ligand Techniques. 1992, Academic Press, pages 110-118. The method can be suitable for the sterilization of solid support material activated with carboxylic acid, amino and hydrazide groups, amongst others.

After sterilization, the pre-activated solid support material can be further modified based on the specific need of the user. For example, for chromatography matrix, ligands for biomolecules of interest can be attached under aseptic conditions, via conventional coupling techniques. The sterile solid support material with aldehyde groups can be further reacted using for example reductive amination. Alternatively, the sterile solid support material with aldehyde groups can be reacted using any molecule containing aldehyde reacting groups, for example hydrazide, hydrazine, hydroxylamine, or semicarbazide.

The sterilization method is widely applicable and may be used for sterilizing solid matrices including chromatography media intended for any purpose, such as the isolation of bioactive molecules including antibodies (particularly monoclonal antibodies), nucleic acids (for example, genomic DNA, RNA), and for the isolation and separation of cells from biological samples. Alternatively, the sterile pre-activated solid matrices provide useful material for the medical device industry, or for certain scavenging applications.

As used herein, the term biological sample refers to a sample obtained from any biological source, including samples of biological tissue or cells obtained harvested *in vivo* or *in situ,* that contains or is suspected of containing nucleic acids or polypeptides such as monoclonal antibodies.

The invention also provides a chromatography matrix sterilized by a method as disclosed above, in particular aldehyde activated agarose matrix. Under aseptic or sterile conditions, ligands can be coupled to the sterile chromatography matrix to generate a sterile chromatographic separation medium.

Other features and advantages of the invention will be apparent from the following examples and from the claims.

### Examples

The present examples are provided for illustrative purposes only, and should not be construed as limiting the invention as defined by the appended claims. All references given below and elsewhere in the present specification are hereby included herein via reference.

The autoclavation was performed using standard procedures for sterilization, 121.1 - 123 degrees for 15 minutes. For each sample, half was used for autoclavation while the other half (the reference flask) was kept in a refrigerator as a control. One extra flask with similar amount of gel and distilled water for temperature log was used.

**Aldehyde SEPHAROSE 4 Fast Flow:** SEPHAROSE 4 Fast Flow (50 ml) was washed 6 times using distilled water. The gel was dried and transferred to a 100 ml E-flask and the total volume adjusted to 75 ml. 0.80 g sodium periodate was added and the oxidation proceeded for 15 minutes. The oxidized gel was washed with distilled water, and then diluted to a total volume of 100 ml. The gel slurry was divided in half (2 x 50 ml) and transferred to Schott flasks. A) for autoclavation B) for reference. After autoclavation of A, both gels were sucked dry and transferred to E-flasks and 0.73 gram n-butylamine in phosphate buffer pH 6.2 (20 ml) was added to each flask, then the volume was adjusted to 50 ml using buffer. 0.170 g NaCNBH₃ was then added to each flask and reactions were stirred overnight.

The reactions were terminated by washing 10 times using water. The Cl ion capacity was measured for both A and B according to standard procedure: The gel was washed 4 times using 0.5 M HCl followed by washing 4 times using 1 mM HCl. 1 ml gel was transferred to a beaker with 10 ml 0.5 M KNO₃ and then diluted to 40 ml using distilled water. After addition of 1 drop HNO₃ conc. the Cl content was determined by potentiometric titration with silver nitrate.

**NHS SEPHAROSE 4 Fast Flow:** NHS SEPHAROSE 4 Fast Flow, 75 ml gel, was washed 6 times with 1 mM HCl. The volume was adjusted to 150 ml using 1mM HCl and dispensed into 3 Schott flasks: C) autoclavation, D) reference and one flask for autoclave temperature measurement. After autoclavation of C the gels were washed with 1 mM HCl and NHS content was measured according to the following protocol: A 1 ml sample was transferred to a 100 ml E-flask and 37 ml 0.1 M ammonium hydroxide was added respectively. Hydrolysis was allowed to proceed for 5 minutes after which the sample was filtered. The absorbance at 260 nm was determined; 0.1 M ammonium hydroxide was used as a blank. NHS content was calculated by using the extinction coefficient, 9700 M⁻¹cm⁻¹.

**CNBr SEPHAROSE 4B:** 15 g freeze dried CNBr SEPHAROSE 4B was swelled for 15 minutes in 75 ml cold 1 mM HCl. After washing 6 times with 1 mM HCl, the volume was adjusted to 100 ml. 50 ml each was dispensed into 2 separate Schott flasks: E) autoclavation and F) reference.

After autoclavation, the gels from both E and F were sucked dry. The gels were transferred, respectively, into 100 ml E-flasks together with 20 ml bicarbonate buffer and 1.3 g 6-AKS dissolved in bicarbonate buffer. Volume was adjusted to 37.5 ml. Reaction was left to proceed for 3 hours. The reaction was terminated by washing 10 times with distilled water. The gel was adjusted to a volume of 37.5 ml in distilled water and samples were taken for titration of carboxylic content: 5 ml gel from E and F were washed five times with 1.0 M KCI. The gel volume was determined and transferred to titer flask and diluted with 10 ml 1.0 M KCl solution. The carboxyl content was determined using NaOH titration.

### Results:

### Aldehyde:

A) autoclaved gave, 20.44 and 20.47 umol/ml gel
B) reference gave, 19.44 and 21.92 umol/ml gel

### NHS:

C) autoclaved gel =0.5 µmol/ml
D) reference gcl 17 µmol/ml.

### CNBr:

E) autoclave gave 1.6 µmol/mol gel
F) reference gave 13.5 µmol/ml gel

These experiments show that both the NHS and CNBr activated agarose gels do not withstand autoclavation. However the aldehyde activated agarose gel does.

## Claims

1. A method for sterilization of an aldehyde activated solid support, the method comprising exposing the solid support to pressurized steam at a temperature of between 121°C and 135°C.

2. The method of claim 1, wherein said solid support is exposed to pressurized steam under a pressure in the range from 2 bars to 35 bars.

3. The method of claim 1, wherein said solid support is exposed to pressurized steam for a time period in the range from 10 to 60 minutes.

4. The method of claim 1, wherein said solid support is exposed to steam at a temperature of 121.1 - 123°C for 15 minutes.

5. The method of claim 1, wherein said solid support to be sterilized is an aldehyde activated chromatography matrix.

6. The method of claim 1, wherein said solid support is an agarose matrix.

7. A method for producing a sterile chromatographic separation medium, comprising coupling a ligand under aseptic conditions to the sterile chromatography matrix produced by the method of claim 5.

## Patentansprüche

1. Verfahren zur Sterilisation eines Aldehyd-aktivierten festen Trägers, wobei das Verfahren umfasst, dass der feste Träger Druckdampf bei einer Temperatur zwischen 121°C und 135 °C ausgesetzt wird.

2. Verfahren nach Anspruch 1, wobei der feste Träger Druckdampf unter einem Druck im Bereich von 2 bar bis 35 bar ausgesetzt wird.

3. Verfahren nach Anspruch 1, wobei der feste Träger Druckdampf für einen Zeitraum im Bereich von 10 bis 60 Minuten ausgesetzt wird.

4. Verfahren nach Anspruch 1, wobei der feste Träger Dampf bei einer Temperatur von 121,1 - 123°C 15 Minuten lang ausgesetzt wird.

5. Verfahren nach Anspruch 1, wobei der zu sterilisierende feste Träger eine Aldehyd-aktivierte Chromatographiematrix ist.

6. Verfahren nach Anspruch 1, wobei der feste Träger eine Agarosematrix ist.

7. Verfahren zur Herstellung eines sterilen chromatographischen Trennmediums, das das Koppeln eines Liganden unter aseptischen Bedingungen an die sterile Chromatographiematrix, hergestellt durch das Verfahren des Anspruchs 5, umfasst.

## Revendications

1. Procédé de stérilisation d'un support solide activé par aldéhyde, le procédé comprenant l'exposition du support solide à de la vapeur pressurisée à une température comprise entre 121°C et 135°C.

2. Procédé selon la revendication 1, dans lequel ledit support solide est exposé à de la vapeur pressurisée sous une pression comprise dans la plage de 2 bars à 35 bars.

3. Procédé selon la revendication 1, dans lequel ledit support solide est exposé à de la vapeur pressurisée pendant une durée comprise dans la plage de 10 à 60 minutes.

4. Procédé selon la revendication 1, dans lequel ledit support solide est exposé à de la vapeur à une température de 121,1 à 123°C pendant 15 minutes.

5. Procédé selon la revendication 1, dans lequel ledit support solide à stériliser est une matrice de chromatographie activée par aldéhyde.

6. Procédé selon la revendication 1, dans lequel ledit support solide est une matrice d'agarose.

7. Procédé de production d'un milieu de séparation de chromatographie stérile, comprenant le couplage d'un ligand sous des conditions aseptiques à la matrice de chromatographie stérile produite par le procédé selon la revendication 5.
